(19) Europäisches Patentamt
European Patent Office
Office européen des brevets

(11) Publication number: 0 341 661 A2

(12) EUROPEAN PATENT APPLICATION

(21) Application number: 89108324.8

(51) Int. Cl.⁴: A61K 31/69 , A61K 37/02

(22) Date of filing: 09.05.89

(30) Priority: 11.05.88 US 192690

(43) Date of publication of application: 15.11.89 Bulletin 89/46

(84) Designated Contracting States: AT BE CH DE ES FR GB GR IT LI LU NL SE

(71) Applicant: E.I. DU PONT DE NEMOURS AND COMPANY
1007 Market Street
Wilmington Delaware 19898(US)

(72) Inventor: Hussain, Munir Alwan
1203 Graylyn Road Chatham
Wilmington Delaware 19803(US)
Inventor: Shenvi, Ashokkumar Bhikkappa
2205 Carlton Lane
Wilmington Delaware 19810(US)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

# (54) Peptide-drug compositions containing alpha-aminoboronic acid derivatives.

(57) Pharmaceutical compositions of peptide or protein drugs are provided which contain $\alpha$-aminoboronic acid derivatives to stabilize these drugs during their absorption through various mucosae, such as nasal and buccal mucosae, and eventually improve their systemic bioavailability.

EP 0 341 661 A2

# Peptide-Drug Compositions Containing α-Aminoboronic Acid Derivatives

## Field of Invention

This invention relates to peptide-drug compositions containing α-aminoboronic acid derivatives, more particularly to such compositions in forms which are administered through various absorptive mucosae.

## Background of the Invention

Peptides and proteins are routinely administered parenterally because of their low bioavailability when administered by other means such as oral, nasal, buccal, and vaginal routes. The fraction of the peptide or protein that is absorbed intact into the systemic circulation depends on how well it resists degradation by peptidases and proteases that are generally found at the site of administration and in the mucosal barrier.

Peptidases are generally responsible for the degradation of peptides in animal tissues ("Mammalian Proteases", A. J. Barrett and J. K. McDonald, Eds., Vol. II, 23-102).

The enzymatic barrier is thought to be very efficient in degrading orally administered peptides, thus leading to poor oral peptide bioavailability. At the same time, this barrier is assumed to be moderate in efficiency at routes of administration other than oral, thereby favoring peptide bioavailability. For example, the delivery of leuprolide, one of the most widely studied peptides, via the nasal, rectal and vaginal routes has been demonstrated to improve its bioavailability by a factor of 3, 35 and 112 times over the oral route, respectively. However, the fraction of peptide absorbed into the systemic circulation is only 0.1, 1.2 and 3.8% of the applied dose respectively. On the other hand, subcutaneous administration of leuprolide resulted in over 65% of the drug being absorbed systemically (Okada et al., J. Pharm. Sci. 71, 1367-1371, 1982). It is possible that this is due to the presence of aminopeptidases in the nasal, rectal and vaginal mucosa (Stratford et al., Int. J. of Pharm. 30, 73-82, 1985). Hydrolysis of leucine enkephalin in the nasal cavity is postulated to be the possible factor in the low bioavailability of nasally administered peptides (A. Hussain, J. Faraj, A. Yukihiko, and J. E. Truelove, Biochem. Biophys. Res. Com. 133, 923, 1985).

Transition state analog inhibitors are molecules which resemble the geometry of a substrate at its transition state and have a much higher affinity for the active site of an enzyme than the substrate itself. Such inhibitors are the aminoboronic acid derivatives described in U.S. Patent No. 4,537,773 and U.S. Patent No. 4,499,082, as potent, reversible inhibitors of certain proteolytic enzymes. These aminoboronic acid derivatives form tetrahedral boronate species from trigonal boronic acid. The tetrahedral boronates resemble the transition state of peptides during their hydrolysis by proteases.

There is a need for effective inhibitors of aminopeptidases to stabilize and improve the delivery of pharmacologically active peptides and proteins by nasal, buccal, rectal and intramuscular administration. The aminoboronic acid derivatives fulfill this need.

## Summary of the Invention

According to the present invention there is provided a pharmaceutical composition consisting essentially of a pharmaceutically suitable carrier, a protein or peptide, optionally an absorption enhancer, and an α-aminoboronic acid derivative of the formula:

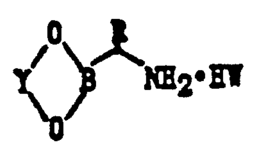

where HW is a mineral acid, sulfonic acid, alkanoic acid or a perfluoroalkanoic acid;
Y is a moiety derived from a dihydroxy compound comprising at least 2 hydroxy groups separated by at least two connecting atoms in a chain or ring, said chain or ring comprising carbon atoms and, optionally, a heteroatom which can be N, S, or O;
R is alkyl or $-CH_2R^1$;
$R^1$ is $-XR^2$, aryl, or aryl substituted with one or more alkyl groups;

X is O or S;
$R^2$ is -H, alkyl, or $-SiR^3R^4R^5$;
$R^3$, $R^4$, and $R^5$ are independently alkyl, aryl, or aryl substituted with one or more alkoxy groups.

The present invention also relates to the use of these effective inhibitors of aminopeptidases to stabilize and improve the delivery of pharmacologically active peptides or proteins by nasal, buccal, rectal, and intramuscular forms of administration.

## Detailed Description of the Invention

(1) By the term "protein or peptide" we mean specifically those peptides which are susceptible to hydrolysis by proteases, specifically aminopeptidases. Preferred proteins or peptides are leucine enkephalin, LH-RH, Tyr-Phe-Met-Arg-Phe-$NH_2$, Tyr-Bradykinin, and Thymopoietin II (TP5).

(2) By the term "absorption enhancers" we mean materials which enhance the uptake of proteins and peptides and can be used to alter penetration characteristics of the drugs. Preferred absorption enhancers are sodium cholate, sodium taurocholate, sodium glycochlate, sodium deoxycholate, sodium taurodeoxycholate and sodium glycodeoxycholate. Other equivalents will be apparent to those skilled in the art.

(3) By the term "pharmaceutically suitable carrier" we mean any non-toxic pharmaceutically suitable vehicle. Suitable carriers include those conventional vehicles used to prepare dosage forms such as capsules, tablets, suppositories, nasal sprays, mucosal patches and adhesives, IUD's, liquid solutions or suspensions. Also, the components, separately or together, can be dissolved in vehicles normally used for injection. These can be formulated using techniques employed by those skilled in the art. Suitable formulations for other routes of administration can be formulated with non-toxic ingredients normally employed by those skilled in the art. It is preferred that a carrier be used which is suitable for administration through absorptive mucosa.

(4) By the term "α-aminoboronic acid derivative" is means any such derivative described in U.S. 4,499,082 and U.S. 4,537,773. Preferred derivatives are those of Examples 5, 13 and 21 of U.S. 4,537,773.

The α-aminoboronic acid derivatives useful in the present invention greatly reduce the hydrolysis of peptides and proteins such as leucine enkephalin, LH-RH, Tyr-Phe-Met-Arg-Phe-$NH_2$ and Tyr-Bradykinin at nanomolar to micromolar concentrations, while much higher concentrations of other known inhibitors, e.g., Bestatin and Puromycin, are needed to exert a similar degree of inhibition.

As mentioned above, delivery of peptides and proteins used in combination with an α-aminoboronic acid derivative can be delivered by several routes. The derivative is used in an amount effective to inhibit hydrolysis of the peptide or protein by proteases and to enhance bioavailability through various mucosae. In the following studies, solutions were used for purposes of testing the delivery of a peptide without the use of an α-aminoboronic acid derivative inhibitor and comparing it to the delivery of the peptide with addition of the inhibitor and an absorption enhancer, if desirable.

Nasal preparations are generally liquid preparations containing the peptide to be delivered with an hydrolysis inhibiting amount of inhibitor and, optionally, an absorption enhancer. Typically, these formulations are made so that approximately 0.1 cc of the liquid containing all of the ingredients can be delivered by a metered spray. The ratio of components on a weight basis is usually 0.0001-10% peptide or protein, 0-10% enhancer, 0.00001 to 5% α-aminoboronic acid derivative inhibitor, 0-10% preservative, and the balance distilled water, saline, or buffer.

Typical buccal formulations contain on a weight basis 0.0001-10% peptide or protein, 0-10% enhancer, 0.00001-5% inhibitor, 0-10% preservative, 0-99.9% non-toxic vehicle, 0-20% suspending agent, and the balance a polymeric matrix, and/or mucosal adhesive.

Typical oral formulations containing the peptide or protein drug, inhibitor and optional enhancer may also include diluents, granulating agents, preservatives, binders, flavoring agents, pigments, and coating agents.

Other formulations, such as suppositories, IUD's, etc., can be formulated using techniques and materials employed by those skilled in the art.

The invention can be further understood by reference to the following examples in which parts and percentages are by weight unless otherwise indicated.

## METHODS OF TESTING THE INHIBITORS

In order to evaluate the effectiveness of the α-aminoboronic acid derivative inhibitors of this invention, an animal model was used (according to A. Hussain, J. Furaj, A. Yukihiko and J. E. Truelove, Biochem. Biophys. Res. Com. 133, 923, 1985) to assess the degradation of proteins and peptides *in situ* and *in vivo in situ* in the nasal cavity of rats.

## Animal Model

The method involved the use of Sprague-Dawley rats weighing approximately 300 grams. Each rat was anesthetized with sodium pentobarbitol (50 mg/kg). An incision was made in the neck and the trachea was canulated with PE 240 tubing. For the *in situ* studies, a second tube, which served to introduce the perfusing solution, was inserted through the esophagus to the posterior part of the nasal cavity. The nasopalatine was closed with an adhesive to prevent drainage of the perfusing solution from the nasal to the oral cavity. For the *in vivo in situ* studies, the esophagus was canulated using PE 240 tubing closed at one end. The closed end was pushed to the posterior end of the nasal passage and then tied in place. The nasopalatine was closed with an adhesive to prevent drainage of the perfusing solution from the nasal to the oral cavity. The rats were dosed in one nostril with 50 µl of peptide solution in two forms; the control solution had no inhibitor added, the test solution had inhibitor added at concentrations specified in the examples shown below. The dosing solution was 0.1 M phosphate buffer (pH 7.4). At certain time intervals (one rat/time interval), the drug was washed from the nose via the other nostril with 10 ml of 0.1 M phosphate buffer (pH 3.8) using a peristaltic pump. The nasal washing was then placed in a 10 ml volumetric flask and adjusted to 10 ml with the same buffer and analyzed for the peptide by HPLC.

The formulations used for the *in vivo in situ* nasal studies contained: the peptide, the inhibitor, 0.1 M pH 7.4 phosphate buffer, purified water, and an enhancer, if desirable. Specific formulations are listed with each example.

## ANALYTICAL METHOD:

The analysis of leucine enkephalin and its major metabolite involved taking aliquots of samples, diluting them with 200 µl of 0.1 M citric acid (pH 2.3) to quench hydrolysis and subjecting the samples to analysis (for *in vivo in situ* studies, the washings were injected as they were obtained). High Pressure Liquid Chromatography (HPLC) was run on a 4.6 x 250 mm $C_8$ column. The mobile phase consisted of 0.1 M $NaH_2PO_4$-$H_3PO_4$-acetonitrile (916 : 5 : 275) and was delivered at a flow rate of 1.7 ml/min. Detection was by UV at 210 nm. Retention times for leucine enkephalin and its major degradation product, Des-Tyr-leucine enkephalin were 7.3 and 4.8 min., respectively. Data were calculated as % of leucine enkephalin remaining in the nasal perfusate as a function of time, counting the concentration at zero time as 100%.

## Example 1

In this example, a 10 ml solution of 0.1 M phosphate buffer solution (pH 7.4) containing 100 µM leucine enkephalin (the peptide) in the absence or presence of 0.1 µM 3-methyl-1(4,4,5,5-tetramethyl-1,3,2-dioxaborolane-2-yl)-1-butamine, trifluoroacetic acid salt (boroleucine) as the inhibitor was perfused nasally in rats as described above.

A 10 ml portion of the peptide solution was placed in a water-jacketed container, maintained at 37° C and circulated through the nasal cavity of rats using a peristaltic pump. The perfusing solution was passed through the nasal cavity, out the nostrils, through a funnel, and returned to the water-jacketed container. The perfusate was analyzed by the ANALYTICAL METHOD described above.

The results obtained from this study are shown in Table 1.

TABLE 1

| IN SITU NASAL DISAPPEARANCE OF 100 µM LEUCINE ENKEPHALIN AND APPEARANCE OF DES-TYR-LEUCINE ENKEPHALIN IN THE NASAL PERFUSATE (10 ml) IN THE ABSENCE AND PRESENCE OF 0.1 µM BOROLEUCINE | | | | | |
|---|---|---|---|---|---|
| O Time | (Min) | No Inhibitor | | 0.1 µM boroleucine | |
| | | % Leucine Enkephalin Remaining | % Des-Tyr-Leucine Enkephalin Forming | % Leucine Enkephalin Remaining | % Des-Tyr-Leucine Enkephalin Forming |
| 1 | 0 | 100.0±0.0 | 0.0±0.0 | 100.0±0.0 | 0.0±0.0 |
| 2 | 5 | 94.3±0.9 | 7.7±0.7 | 96.6±1.5 | 0.6±0.2 |
| 3 | 10 | 90.5±1.5 | 12.5±1.1 | 93.5±3.0 | 0.9±0.3 |
| 4 | 20 | 79.9±6.8 | 23.0±2.9 | 96.4±1.7 | 1.3±0.6 |
| 5 | 30 | 66.0±7.6 | 31.3±4.4 | 92.7±3.3 | 1.4±0.2 |
| 6 | 45 | 53.4±6.7 | 44.0±6.8 | 91.8±2.5 | 1.9±0.2 |
| 7 | 60 | 40.0±6.7 | 51.8±5.9 | 88.8±2.6 | 2.4±0.3 |
| 8 | 90 | 21.5±7.1 | 58.4±3.3 | 85.6±3.5 | 3.4±0.5 |

In the absence of the inhibitor, about 58% of the degradation product, Des-Tyr-Leucine Enkephalin, was formed after circulating the perfusate through the nose for 90 min. On the other hand, only about 3% of this degradation product was formed in the presence of inhibitor.

## Example 2

In this example, a 10 ml solution of 0.1 M phosphate buffer (pH 7.4) containing 100 µM leucine enkephalin in the absence of inhibitor and in the presence of 0.01, 0.03, or 0.1 µM boroleucine was perfused nasally in rats in a fashion similar to that of Example 1. The perfusate was analyzed by HPLC as described in Example 1 and analyzed for degradation of the leucine enkephalin. The results obtained are shown below in Table 2.

TABLE 2

IN SITU NASAL DEGRADATION OF 100 μM LEUCINE ENKEPHALIN IN THE NASAL PERFUSATE (10 ml) IN THE ABSENCE OF BOROLEUCINE AND IN THE PRESENCE OF 0.01 MICRO M BOROLEUCINE, 0.03 MICRO M BOROLEUCINE AND 0.1 MICRO M BOROLEUCINE

| Time (Min) | No Inhibitor | 0.01 Micro M Boroleucine | 0.03 Micro Boroleucine | 0.1 Micro M Boroleucine |
|---|---|---|---|---|
| 0 | 100.0±0.0 | 100.0±0.0 | 100.0±0.0 | 100.0±0.0 |
| 5 | 94.3±0.9 | | 94.1±1.7 | 96.6±1.5 |
| 10 | 90.5±1.5 | 90.6±3.5 | 93.6±1.6 | 93.5±3.0 |
| 20 | 79.9±6.8 | 89.1±5.3 | 91.8±2.2 | 96.4±1.7 |
| 30 | 66.0±7.6 | 79.1±2.9 | 88.3±1.4 | 92.7±3.3 |
| 45 | 53.4±6.7 | 74.9±2.8 | 85.7±1.3 | 91.8±2.5 |
| 60 | 40.0±6.7 | 69.2±2.0 | 81.5±1.4 | 88.8±2.6 |
| 90 | 21.6±7.1 | 56.2±1.6 | 72.9±1.0 | 85.6±3.5 |

The data show that increasing concentrations of the boroleucine inhibitor causes an increased inhibition of degradation of leucine enkephalin and, thus, higher concentrations of the leucine enkephalin are found in the perfusate as a function of time.

Example 3

In this example, a 0.1 M phosphate buffer solution (pH 7.4) containing 100 μM leucine enkephalin in the absence of inhibitor and in the presence of the inhibitors 0.1 μM boroleucine, or 0.1 μM 4,4,5,5-tetramethyl-α-(1-methylethyl)-1,3,2-dioxaborolane-2-methanamine, trifluoroacetic acid salt (borovaline), or 0.1 μM α,4,4,5,5-pentamethyl-1,3,2-dioxaborolane-2-methanamine, trifluoroacetic acid salt (boroalanine) were perfused nasally in rats as described in Example 1. The analysis of the degradation of leucine enkephalin was followed by HPLC as described in Example 1. The data obtained are shown below in Table 3.

TABLE 3

IN SITU NASAL DEGRADATION OF 100 μM LEUCINE ENKEPHALIN IN THE NASAL PERFUSATE (10 ml) IN THE ABSENCE OF INHIBITOR AND IN THE PRESENCE OF 0.1 MICRO M BOROLEUCINE, 0.1 MICRO M BOROVALINE AND 0.1 MICRO M BOROALANINE

| Time (Min) | No Inhibitor | 0.1 Micro M Boroleucine | 0.1 Micro M Borovaline | 0.1 Micro M Boroalanine |
|---|---|---|---|---|
| 0 | 100.0±0.0 | 100.0±0.0 | 100.0±0.0 | 100.0± 0.0 |
| 5 | 94.3±0.9 | 96.6±1.5 | 96.3±1.9 | 94.7± 3.0 |
| 10 | 90.5±1.5 | 93.5±3.0 | 95.7±1.5 | 91.9± 2.1 |
| 20 | 79.9±6.8 | 96.4±1.7 | 94.8±1.4 | 88.5± 2.2 |
| 30 | 66.0±7.6 | 92.7±3.3 | 91.6±1.6 | 84.1± 3.1 |
| 45 | 53.4±6.7 | 91.8±2.5 | 89.1±2.5 | 77.5± 4.4 |
| 60 | 40.0±6.7 | 88.8±2.6 | 86.2±2.5 | 69.9± 6.7 |
| 90 | 21.6±7.1 | 85.6±3.5 | 78.3±4.3 | 56.4±12.1 |

The data in this table show a comparison of the inhibitory effect of equimolar concentrations of three α-aminoboronic acid derivatives against leucine enkephalin. The inhibitors, boroleucine and borovaline, are superior to boroalanine.

## Example 4

In this example, a 0.1 M phosphate buffer solution (pH 7.4) containing 100 μM of the leucine enkephalin in the absence of inhibitor and in the presence of known inhibitors (10 μM bestatin, or 100 μM puromycin), were compared versus the presence of 0.1 μM boroleucine. The solutions were nasally perfused in rats according to the procedure described in Example 1 and analyzed by HPLC as described in Example 1.

The data obtained are shown below in Table 4.

TABLE 4

| IN SITU NASAL DEGRADATION OF 100 µM LEUCINE ENKEPHALIN IN THE NASAL PERFUSATE (10 ml) IN THE ABSENCE OF INHIBITOR AND IN THE PRESENCE OF 10 µM BESTATIN, 100 µM PUROMYCIN, AND 0.1 µM BOROLEUCINE | | | | |
|---|---|---|---|---|
| Time (Min) | No Inhibitor | 10 µM Bestatin | 100 µM Puromycin | 0.01 µM Boroleucine |
| 0 | 100.0±0.0 | 100.0±0.0 | 100.0±0.0 | 100.0±0.0 |
| 5 | 94.2±0.9 | 97.3±1.6 | 96.7±2.4 | 96.6±1.5 |
| 10 | 90.5±1.5 | 94.7±1.7 | 91.3±3.4 | 93.5±3.0 |
| 20 | 79.9±6.8 | 90.0±3.3 | 82.4±4.1 | 96.4±1.7 |
| 30 | 66.0±7.6 | 86.6±3.8 | 78.5±3.2 | 92.7±3.3 |
| 45 | 53.4±6.7 | 79.0±5.3 | 73.6±2.2 | 91.8±2.5 |
| 60 | 40.0±6.7 | 72.3±6.7 | 63.5±5.6 | 88.8±2.6 |
| 90 | 21.5±7.0 | 57.3±8.8 | 48.9±6.7 | 85.7±3.5 |

The data in the above table show a comparison of the in situ degradation of the peptide leucine enkephalin, in the absence of inhibitors and in the presence of three different inhibitors administered at different concentrations. The inhibition of degradation was in the following order boroleucine> bestatin> puromycin. The inhibitory effect of boroleucine was better than bestatin at molar concentrations that are 1/100th the concentration of bestatin. This clearly shows the potency of boroleucine as an inhibitor versus currently known inhibitors.

Example 5

In this example, a 0.1 M phosphate buffer solution (pH 7.4) containing 35 µM LH-RH (fragment 3-10) in the absence of inhibitor and in the presence of the inhibitor, boroleucine at a concentration of 0.46 µM was administered to rats as described in Example 1. The perfusate was analyzed by HPLC as described in Example 1 with the exception of the mobile phase used. The mobile phase was comprised of 0.1 M $NaH_2PO_4$:$H_3PO_4$:$CH_3CN$(916:5:240) delivered at 1.7 ml/min and analyzed by UV at 210 nm. The data obtained are shown in Table 5.

TABLE 5

| IN SITU NASAL DISAPPEARANCE OF 35 µM LH (FRAGMENT 3-10) IN THE NASAL PERFUSATE (10 ml) IN THE ABSENCE AND PRESENCE OF 0.46 µM BOROLEUCINE | | |
|---|---|---|
| Time (Min) | Absence of Boroleucine | 0.465 µM Boroleucine |
| 0 | 100.0 | 100.0 |
| 5 | 96.0 | 96.7 |
| 10 | 93.8 | 97.5 |
| 20 | 91.6 | 95.5 |
| 30 | 85.3 | 96.5 |
| 45 | 70.4 | 95.8 |
| 60 | 61.3 | 91.9 |
| 90 | 45.0 | 92.9 |
| 120 | 24.4 | 86.4 |

The disappearance of LH-RH is much faster in the absence of inhibitor. The presence of boroleucine in very small concentration allowed for retention of about 86% of the compound as compared to 24% without the inhibitor.

## Example 6

In this example, a 0.1 M phosphate buffer solution (pH 7.4) containing 100 µM Tyr-Phe-Met-Arg-Phe-$NH_2$ was administered nasally to rats as described in Example 1. The peptide was administered in the absence of inhibitor and in the presence of 2 µM boroleucine. The perfusate was analyzed by HPLC using the procedure described in Example 1 with the exception that the mobile phase was comprised of 0.1 M $NaH_2PO_4$:$H_3PO_4$:$CH_3CN$ (200:1:60) delivered at 1.4 ml/min. Detection was by UV at 210 nm.

The data obtained are shown in Table 6.

TABLE 6

| IN SITU NASAL DISAPPEARANCE OF 100 μM TYR-PHE-ARG-PHE-$NH_2$ IN THE NASAL PERFUSATE (10 ml) IN THE ABSENCE OF BOROLEUCINE AND IN THE PRESENCE OF 2 μM BOROLEUCINE | | |
|---|---|---|
| Time (Min) | Absence of Boroleucine | 2 μM Boroleucine |
| 0 | 100.0 | 100.0 |
| 5 | 85.4 | 93.8 |
| 10 | 80.7 | |
| 20 | 71.1 | 84.3 |
| 30 | 59.5 | 78.8 |
| 45 | 44.4 | 70.5 |
| 60 | 30.4 | 63.3 |
| 90 | 10.6 | 49.5 |
| 120 | 2.8 | 32.9 |

The data above show that at a concentration of 2 μM boroleucine, 33% of the peptide remained intact as compared to 3% without the inhibitor present.

## Example 7

In this example, a 0.1 M phosphate buffer solution (pH 7.4) containing (100 μg/rat) leucine enkephalin was administered to rats with 0.05% sodium cholate as a penetration enhancer. The peptide and enhancer were administered without an inhibitor and with boroleucine, as the inhibitor, present at a concentration of 0.054 μg/rat. The animal model for this study remained the same as that described in Example 1 with the exception of the dosing and sampling regime. The method of administration involved dosing the rats in one nostril with 50 μl of the peptide solution with or without the inhibitor by using a 50 μl Hamilton syringe with 1/2 cm of PE-50 tubing attached to the end. At certain time intervals (one rat/interval), the drug was washed from the nose via the other nostril with 10 ml of 0.1 M phosphate buffer (pH 3.8) using a peristaltic pump. The nasal washing was then placed in a 10 ml volumetric flask and adjusted to 10 ml with the same buffer and analyzed for the peptide by using the same analytical method described in Example 1.

The data generated for this example are shown in Table 7.

TABLE 7

| IN VIVO/IN SITU NASAL DISAPPEARANCE OF LEUCINE ENKEPHALIN (100 µg/RAT) AND APPEARANCE OF DES-TYR-LEUCINE ENKEPHALIN IN THE PRESENCE OF 0.05% NA CHOLATE WITH AND WITHOUT BOROLEUCINE (0.054 µg/RAT) | | | | |
|---|---|---|---|---|
| Time (Min) | Leu Enk No Inhibitor | Des-Tyr No Inhibitor | Leu Enk w/Inhibitor | Des-Tyr w/Inhibitor |
| 0 | 92.1±6.9 | | 94.4± 7.2 | |
| 5 | 54.0±5.4 | 17.0±2.7 | 71.4± 1.8 | |
| 10 | 34.7±9.2 | 21.5±1.2 | 64.3± 5.6 | 0.6±0.4 |
| 20 | 19.8±9.2 | 21.0±4.1 | 38.1±15.2 | 1.6±0.7 |
| 30 | 7.6±6.2 | 16.5±3.8 | 28.7±10.8 | 1.9±1.1 |
| 50 | 2.3±1.2 | 7.9±2.2 | 12.8± 4.0 | 2.2±0.4 |

The in vivo, in situ nasal disappearance of leucine enkephalin and the appearance of its major metabolite, Des-Tyr-leucine enkephalin was followed. In the absence of inhibitor, the leucine enkephalin disappearance was via absorption and degradation to Des-Tyr-leucine enkephalin and further absorption of the latter. While in the presence of inhibitor, the disappearance of leucine enkephalin was primarily due to absorption and negligible degradation was observed.

## Example 8

Example 7 was followed except the peptide studied was Tyr-Phe-Met-Arg-Phe-$NH_2$. In this case, the peptide was administered at a concentration of 50 µg in a 50 µl volume/rat with or without the inhibitor boroleucine. The concentration used for the inhibitor was 0.3 µg/rat. In this study, no enhancer was employed.

The data generated are shown in Table 8.

TABLE 8

| IN SITU/IN VIVO NASAL DISAPPEARANCE OF TYR-PHE-MET-ARG-PHE-$NH_2$ 50 (µg/RAT) IN THE ABSENCE AND PRESENCE OF BOROLEUCINE (0.3 µg/RAT) | | |
|---|---|---|
| Time (Min) | Absence of Boroleucine | 0.3 µg/Rat Boroleucine |
| 0.0 | 100.0 ± 0.0 | 100.0 ± 0.0 |
| 2.5 | 60.4 ± 8.5 | 84.2 ± 3.8 |
| 5.0 | 39.9 ± 5.2 | 67.3 ± 1.7 |
| 10.0 | 21.5 ± 3.9 | 42.1 ± 6.7 |

The results show that the disappearance of Tyr-Phe-Met-Arg-Phe-$NH_2$ was faster in the absence of inhibitor.

## Claims

1. A pharmaceutical composition consisting essentially of a pharmaceutically suitable carrier, a protein or peptide, optionally an absorption enhancer, and an α-aminoboronic acid derivative of the formula:

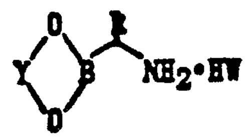

where HW is a mineral acid, sulfonic acid, alkanoic acid or a perfluoroalkanoic acid;
Y is a moiety derived from a dihydroxy compound comprising at least 2 hydroxy groups separated by at least two connecting atoms in a chain or ring, said chain or ring comprising carbon atoms and, optionally, a heteroatom which can be N, S, or O;
R is alkyl or $-CH_2R^1$;
$R^1$ is $-XR^2$, aryl, or aryl substituted with one or more alkyl groups;
X is O or S;
$R^2$ is -H, alkyl, or $-SiR^3R^4R^5$;
$R^3$, $R^4$, and $R^5$ are independently alkyl, aryl, or aryl substituted with one or more alkoxy groups.

2. A pharmaceutical composition of Claim 1 wherein the protein or peptide is one susceptible to hydrolysis by proteases.

3. A pharmaceutical composition of Claim 2 wherein the protein or peptide is selected from the group consisting of leucine enkephalin, LH-RH, Tyr- Phe-Met-Arg-Phe-$NH_2$, Tyr-Bradykinin, and Thymopoietin II.

4. A pharmaceutical composition of Claim 1 wherein Y is $(CH_3)_2\underset{|}{C}-\underset{|}{C}(CH_3)_2$,

$-CH_2CH_2-$, $(CF_3)_2\underset{|}{C}-\underset{|}{C}(CF_3)_2$,

or $-(CH_2)_2NH(CH_2)_2-$.

5. A pharmaceutical composition of Claim 1 wherein R is $-CH_3$, $-CH(CH_3)_2$, $-CH_2CH_2(CH_3)_2$, $-CH(CH_3)-C_2H_5$, or

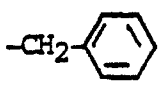 .

6. A pharmaceutical composition of Claim 1 wherein HW is $CF_3CO_2H$, $CH_3CO_2H$, HCl, or HBr.

7. A pharmaceutical composition of Claim 1 wherein the α-aminoboronic acid derivative is 3-methyl-1-(4,4,5,5-tetramethyl-1,3,2-dioxaborolane-2-yl)-1-butamine, trifluoroacetic acid salt.

8. A pharmaceutical composition of Claim 1 in a dosage form suitable for buccal administration.

9. A pharmaceutical composition of Claim 1 in a dosage form suitable for nasal administration.

10. A method of administering a peptide or protein drug susceptible to hydrolysis by proteases which comprises administering the drug in the presence of an α-aminoboronic acid derivative of the formula:

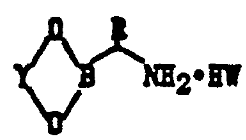

where HW is a mineral acid, sulfonic acid, alkanoic acid or a perfluoroalkanoic acid;
Y is a moiety derived from a dihydroxy compound comprising at least 2 hydroxy groups separated by at least two connecting atoms in a chain or ring, said chain or ring comprising carbon atoms and, optionally, a heteroatom which can be N, S, or O;
R is alkyl or $-CH_2R^1$;
$R^1$ is $-XR^2$, aryl, or aryl substituted with one or more alkyl groups;
X is O or S;
$R^2$ is -H, alkyl, or $-SiR^3R^4R^5$;

$R^3$, $R^4$, and $R^5$ are independently alkyl, aryl, or aryl substituted with one or more alkoxy groups.

11. The method of Claim 10 wherein the protein or peptide is is selected from the group consisting of leucine enkephalin, LH-RH, Tyr-Phe-Met-Arg-Phe-$NH_2$, Tyr-Bradykinin, and Thymopoietin II.

12. The method of Claim 10 wherein the peptide or protein is administered through absorptive mucosa.